(19) Europäisches Patentamt / European Patent Office / Office européen des brevets

(11) **EP 3 614 903 B1**

(12) **EUROPÄISCHE PATENTSCHRIFT**

(45) Veröffentlichungstag und Bekanntmachung des Hinweises auf die Patenterteilung:
**26.03.2025   Patentblatt 2025/13**

(21) Anmeldenummer: **18719197.8**

(22) Anmeldetag: **26.04.2018**

(51) Internationale Patentklassifikation (IPC):
***A61B 3/00*** *(2006.01)*

(52) Gemeinsame Patentklassifikation (CPC):
**A61B 3/0008; A61B 90/30;** A61B 2090/309

(86) Internationale Anmeldenummer:
**PCT/EP2018/060817**

(87) Internationale Veröffentlichungsnummer:
**WO 2018/197651 (01.11.2018 Gazette 2018/44)**

(54) **VORRICHTUNG ZUR BELEUCHTUNG DES INTRAOKULAREN RAUMES**

DEVICE FOR LIGHTING THE INTRAOCULAR AREA

DISPOSITIF D'ÉCLAIRAGE DE L'ESPACE INTRAOCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:   26.04.2017   EP 17168314
27.04.2017   EP 17168571

(43) Veröffentlichungstag der Anmeldung:
**04.03.2020   Patentblatt 2020/10**

(73) Patentinhaber: PHARMPUR GmbH
**86343 Königsbrunn (DE)**

(72) Erfinder:
• **LINGENFELDER, Christian**
**89081 Ulm (DE)**
• **HESSLING, Martin**
**89075 Ulm (DE)**
• **KÖLBL, Philipp**
**88239 Wangen im Allgäu (DE)**

(74) Vertreter: **Charrier Rapp & Liebau**
**Patentanwälte PartG mbB**
**Fuggerstraße 20**
**86150 Augsburg (DE)**

(56) Entgegenhaltungen:
EP-B1- 2 060 226          WO-A1-2011/019505
DE-A1- 102006 051 736     US-A1- 2004 004 846
US-A1- 2008 208 176       US-A1- 2013 038 836
US-A1- 2014 288 417

**Beschreibung**

**Gebiet** der Erfindung

Die Erfindung betrifft eine Vorrichtung zur Illumination des Auges gemäß dem Oberbegriff des Anspruchs 1.

Hintergrund der Erfindung

Endoilluminatoren zur Ausleuchtung des Auges sind bekannt, weisen jedoch Nachteile auf. So ist z.B. aus US 2004/0004846 A1 eine Vorrichtung bekannt, die in einem Gehäuse eine LED als Lichtquelle aufweist, wobei diese Lichtquelle Licht in eine Lichtfaser einspeist, die dann das Auge ausleuchtet. Nachteil einer derartigen Vorrichtung ist es, dass in solchen Vorrichtungen das Licht nur sehr ineffizient von der Lichtquelle zum zu beleuchtenden Ort gebracht wird, sodass am vorgesehenen Ort nur etwa 1% der ursprünglichen Lichtleistung ankommt.

Obwohl eine gute Ausleuchtung des Auges bzw. des Augenhintergrundes notwendig ist, muss auch darauf geachtet werden, dass keine phototoxischen Schäden hervorgerufen werden. Eine gute Ausleuchtung basiert auf der Farbtemperatur, der Abstrahlcharakteristik und der Helligkeit des Emitters im Auge. Aus diesen Faktoren ergibt sich die Leistung, welche auf einer gewissen Fläche des Augenhintergrunds ankommt. Helles und energiereiches Licht weist eine hohe Leistung auf und kann Schäden an der Netzhaut, den Photorezeptoren und dem Sehnerv hervorrufen. So ist zwar bläuliches Licht für Operationen bevorzugt, da die Umgebung im Auge rötlich ist und blaues Licht eine bessere Wahrnehmung der Kontraste ermöglicht, bläuliches Licht ist jedoch energiereicheres Licht und kann daher eher zu Schäden führen. Mit anderen Worten ist daher eine sehr gute Ausleuchtung eines Operationsbereiches erwünscht, zugleich soll jedoch die Ausleuchtung möglichst schonend für das Auge im Hinblick auf die Art der eingebrachten Strahlung, welche eine Belastung für das Gewebe darstellen kann, aber auch im Hinblick auf das Ausmaß der Intervention am Auge erfolgen. Eine hohe Strahlenbelastung entsteht insbesondere, wenn die Lichtquelle zu nahe an die Netzhaut kommt und/oder wenn die Beleuchtung zu lange dauert. Die DIN-Norm ISO/DIS15004-2 zeigt die Anforderungen für Behandlungen am und im Auge. Wichtig ist daher, dass nicht nur die Lichtquelle sondern auch deren Abstand zur Netzhaut in jedem Fall definiert ist, da die Strahlungsleistung der Lichtquelle auf eine definierte Fläche der Netzhaut umso höher wird, je näher sie der Netzhaut ist. Dies entspricht dem Abstands-Quadrat-Gesetz und sagt in anderen Worten, dass die Leistung welche auf eine Fläche A mit kleiner werdendem Abstand fällt, sich quadratisch erhöht.

Abstands-Quadrat-Gesetz:

$$E \sim \frac{1}{s^2}$$

| | | |
|---|---|---|
| *E* | irradiance | W cm$^{-2}$ |
| s | working distance | mm |

Die DE 10 2006 051 736 A1 offenbart ein medizinisches Handgerät zur Beleuchtung, insbesondere zur Anwendung in der Ophthalmologie, mit einem als Griffstück dienenden Gehäuse, einer sich vom Gehäuse aus ersteckenden Sonde und einem endseitigen Lichtauslass, sowie einer in das Handgerät integrierten Lichtquelle, wobei die Lichtquelle in dem Gehäuse angeordnet ist und das von der Lichtquelle emittierte Licht über eine sich durch die Sonde erstreckende Lichtleitfaser zum Lichtauslass gelangt.

Die EP 2 060 226 B1 offenbart einen Endoilluminator mit einem stabförmigen oder röhrenförmigen Körper, der ein proximales und ein distales Ende aufweist und in eine kleindimensionierte Öffnung einführbar ist, wobei der Endoilluminator einen LED-Chip umfasst und der Körper einen distalen, hohlen Abschnitt, der vor dem distalen Ende beginnt und bis zum distalen Ende reicht, sowie einen Zuleitungsabschnitt, der am proximalen Ende beginnt und bis zum distalen hohlen Abschnitt reicht, aufweist und der LED-Chip im distalen hohlen Abschnitt des stabförmigen oder röhrenförmigen Körpers angeordnet ist und über den Zuleitungsabschnitt mit Energie versorgt wird. Zwischen dem LED-Chip und dem distalen Ende des Körpers oder am distalen Ende des Körpers ist dabei ein Konverterleuchtstoff angeordnet, dessen Konvertereigenschaften hinsichtlich des von dem LED-Chip emittierten Lichts derart ausgewählt ist, dass er das von dem LED-Chip emittierte Licht in Licht mit einer gewünschten Wellenlängenverteilung konvertiert. Der distale hohle Abschnitt des Körpers ist an seinem distalen Ende von einer wenigstens im sichtbaren Spektralbereich durchlässigen Scheibe abgeschlossen.

Aus der US 2008/0208176 A1 ist eine Vorrichtung zum Injizieren einer ophthalmologischen Vorrichtung in ein Auge bekannt, welche einen hohlzylindrischen Körper und einen darin einführbaren Kolben aufweist, wobei der Kolben in einer Ausführungsform eine bspw. als LED ausgebildete Lichtquelle, die an einer distalen Endfläche des Kolbens befestigt ist, umfasst.

Endoilluminatoren werden generell in der Ophthalmochirurgie und insbesondere in der Pars plana Vitrektomie eingesetzt, um den intraokularen Raum auszuleuchten. Die Nachteile der Endoilluminatoren aus dem Stand der Technik sind insbesondere in der entweder zu geringen Ausleuchtung oder in der hohen, weil kaum kontrollierbaren Strahlenbelastung des Auges zu sehen.

Es war daher eine Aufgabe der Erfindung, eine Vorrichtung zur Illumination für die Ophthalmochirurgie, z.B. Pars plana Vitrektomie, bereitzustellen, die das Operationsgebiet gezielt und optimal ausleuchten kann und die empfindlichen Bereiche des Auges, insbesondere die Netzhaut bestmöglich schützt. Weiterhin war es eine Aufgabe der Erfindung, eine Vorrichtung bereitzustellen, die flexibel einstellbar ist, bei der z.B. Farbe, Intensität, Richtung und Abstrahlwinkel des Lichts angepasst werden können und die ein Ausleuchten mit unterschiedlichen Wellenlängen bzw. aus einer Kombination von Wellenlängen ermöglicht. Weiterhin war es Aufgabe der Erfindung, eine Vorrichtung bereitzustellen, bei der die emittierte Strahlung der Lichtquelle im Rahmen von gesetzlichen oder durch Vorschriften geregelten Grenzwerten liegt. Eine weitere Aufgabe der Erfindung war es, eine Vorrichtung bereitzustellen, die kompakt ist und auch unter schwierigen äußeren Umständen zur Analytik im Auge verwendet werden kann.

Diese Aufgaben werden gelöst durch die Vorrichtung gemäß Anspruch 1. Insbesondere wird eine Vorrichtung zur gezielten Beleuchtung eines intraokularen Raumes eines menschlichen oder tierischen Auges zur Verfügung gestellt, die sehr flexibel einsetzbar ist und in einfacher Weise verwendet werden kann. Die Vorrichtung kann dem Bedarf angepasst werden. Darüber hinaus bietet die erfindungsgemäße Vorrichtung den Vorteil, dass sie nicht nur auf den jeweiligen Anwendungszweck und für die jeweiligen Erfordernisse einstellbar ist, sondern dass auch für den Nutzer die Möglichkeit besteht, während der Verwendung Anpassungen vorzunehmen. Insbesondere können mit der erfindungsgemäßen Vorrichtung Parameter wie Strahlungsstärke, Farbe beleuchteten Fläche für den jeweiligen Einsatz angepasst werden. Weiterhin können verschiedene Parameter der Strahlung/des Licht auch während der Nutzung nach Wunsch verändert werden. Die Vorrichtung umfasst einen Sockel, der an seinem proximalen Ende einen Schaft und an seinem distalen Ende eine Sonde aufweist. Die Sonde ist mit einer Lichtquelle versehen, die wiederum mit einer Energiequelle verbunden ist. Die Lichtquelle weist mindestens eine LED auf, kann aber auch mehrere LEDs haben, um z.B. einen gewünschten Farbwert einzustellen. Wesentlich für die vorliegende Erfindung ist es, dass die Lichtquelle am distalen Ende der Sonde positioniert ist.

Weitere vorteilhafte Ausführungsformen finden sich in den Unteransprüchen.

## Figuren

Figur 1 zeigt eine Skizze einer Ausführungsform der erfindungsgemäßen Vorrichtung zur gezielten Ausleuchtung des intraokularen Raumes eines menschlichen oder tierischen Auges.

Figur 2 zeigt eine Skizze einer weiteren Ausführungsform der erfindungsgemäßen Vorrichtung zur gezielten Ausleuchtung des intraokularen Raumes eines menschlichen oder tierischen Auges.

Figur 3 zeigt eine Skizze einer weiteren Ausführungsform des erfindungsgemäßen Ophthalmoilluminators.

Figur 4 zeigt eine Skizze des distalen Bereichs des erfindungsgemäßen Ophthalmoilluminators.

Figur 5 zeigt eine Skizze einer für den Einsatz in der erfindungsgemäßen Vorrichtung geeigneten LED.

Figur 6A stellt die Bestrahlungsstärken einer erfindungsgemäßen LED bei 10 mA in Abhängigkeit von der Wellenlänge des emittierten Lichtes bei 0 mm Abstand zur Detektionsfläche dar.

Figur 6B zeigt die Bestrahlungsstärke $E_{A-R}$ mit der Auswertefunktion $A(\lambda)$, maximale Belichtungszeit $t_{max}$ ($E_{A-R}$), die Bestrahlungsstärke $E_{VIS-R}$ unter Verwendung der Auswertefunktion $R(\lambda)$ und die Bestrahlungsstärke ohne Bewertungsfunktion ($E_{300-850}$) dargestellt.

Figur 7 zeigt Bilder eines Beleuchtungstests mit einem erfindungsgemäßen Ophthalmoilluminator an einem porkinen Auge.

## Bezugszeichenliste

1   Vorrichtung
2   Sockel
3   Schaft

4 Sonde
5 Lichtquelle
6 Energiequelle
21 Abstandshalter
22 distales Ende des Sockels
23 proximales Ende des Sockels
24 Kanal
30 Halterung
61 Leitung
62 Strombegrenzer
70 LED-Stift
72 Sockel LED-Stift
73 Schaft LED-Stift
74 Sonde LED-Stift
75 Lichtquelle LED-Stift
76 Schalter LED-Stift
80 Trokar
81 Halteteil Trokar
82 Kanüle Trokar

## Definitionen

Die Bezeichnung "intraokularer Raum", wie sie in der vorliegenden Beschreibung verwendet wird, bezeichnet den gesamten Innenraum des Auges, insbesondere hinter der Sklera und schließt den Augenhintergrund mit ein.

Der Ausdruck "Sockel", wie er hier verwendet wird, betrifft eine Halterung, die auf einer Seite zur Aufnahme einer Sonde und auf der anderen Seite zur Aufnahme eines Schafts geeignet ist.

Der Ausdruck "verbindbar", wie er hier verwendet wird, bedeutet, dass zwei Teile miteinander verbindbar oder verbunden sind. Jede Art von Verbindung ist geeignet, wobei eine dauerhafte ebenso wie eine vorübergehende Fixierung eingeschlossen ist. Verbindbar bedeutet insbesondere, dass ein Teil mit einem anderen zeitweise verbunden werden kann und gegebenenfalls durch ein anderes Teil ausgetauscht werden kann.

Der Ausdruck "integral" bedeutet zu einem Ganzen gehören bzw. ein Ganzes bilden, d.h. Teile oder Geräte, die als integral bezeichnet werden, bilden eine Einheit.

Der Ausdruck "Sonde" bezeichnet eine Vorrichtung, die stabförmig ist und innen mindestens einen Kanal aufweist.

Erfindungsgemäß wird eine Lichtquelle mit mindestens einer LED verwendet. Der Begriff "LED" umfasst jede Art von LED, wie sie im Stand der Technik bekannt ist, d.h. insbesondere LED und OLED. Der Begriff "LED" schließt LEDs geringer Größe, z.B. Mikro-LEDs oder Nano-LEDs ein. Der Begriff LED schließt LEDs jeder Wellenlänge ein, d.h. LEDs, die Licht jeder Wellenlänge von UV bis zu fernem Infrarot und darüber hinaus abstrahlen, ein.

Unter dem Begriff "Licht im sichtbaren Bereich" wird Strahlung mit einer Wellenlänge von etwa 0,38 bis etwa 0,78 $\mu$m.

Unter dem Begriff "Licht mit einer Wellenlänge im nahen Infrarot" wird Infrarotstrahlung mit einer Wellenlänge von etwa 0,78 bis etwa 2,5 $\mu$m und einer Wellenzahl von etwa 12500 bis etwa 4000 cm$^{-1}$ verstanden.

Unter dem Begriff "Licht mit einer Wellenlänge im mittleren Infrarot" wird Infrarotstrahlung mit einer Wellenlänge von etwa 2,5 bis etwa 25 $\mu$m und einer Wellenzahl von etwa 4000 bis etwa 400 cm$^{-1}$ verstanden.

Unter dem Begriff "Licht mit einer Wellenlänge im fernen Infrarot" wird Infrarotstrahlung mit einer Wellenlänge von etwa 25 bis etwa 1000 $\mu$m und einer Wellenzahl von etwa 400 bis etwa 10 cm$^{-1}$ verstanden.

Unter dem Begriff "Licht mit einer Wellenlänge im UV" wird UV-Strahlung mit einer Wellenlänge von etwa 0,2 bis etwa 0,38 $\mu$m verstanden.

Die Begriffe "Strahlung" und "Licht" werden hier in gleicher Weise verwendet. Wenn der Begriff Licht verwendet wird, so soll Strahlung einer Wellenlänge von UV bis fernem Infrarot umfasst sein. Entsprechend bezieht sich der Begriff Lichtquelle auf eine Quelle, die Strahlung mit einer Wellenlänge im Bereich von UV bis fernem IR abgibt.

Der Begriff "Lichtquelle mit mindestens einer LED" bezeichnet solche Lichtquellen, die mindestens eine LED aufweisen, aber auch mehrere LEDs in Kombination aufweisen können. Insbesondere fallen unter diesen Begriff LEDs in allen geeigneten Ausführungsformen sowie Kombinationen von LEDs, die verwendet werden, um eine gewünschte Farbe zu erzeugen, insbesondere RGB-LEDs. Außerdem fallen unter diesen Begriff auch Lichtquellen, die mindestens eine LED, die Infrarotstrahlung, sichtbares Licht oder UV-Strahlung abstrahlt, oder eine Kombination davon aufweisen, z.B. Lichtquellen, die eine Kombination von LEDs, die Licht im sichtbaren Bereich (etwa 0,38 bis 0,78 $\mu$m) und solche, die Licht im unsichtbaren, d.h. Infrarot- oder UV-Bereich abstrahlen, umfassen.

Eine "Energiequelle", wie sie hier verstanden wird, ist jede Vorrichtung, die der erfindungsgemäßen Lichtquelle Energie, insbesondere Strom, zuführen kann, um sie zum Leuchten zu bringen. Eine Energiequelle ist insbesondere ein Element, das die für den Betrieb der Vorrichtung adäquate Menge an Strom liefern kann, um die Lichtquelle kontrolliert zum Leuchten zu bringen. Beispiele für Energiequellen sind Akkus, Batterien, Kondensatoren etc.

In einer Ausführungsform hat die erfindungsgemäße Vorrichtung die Form eines Stifts und wird in dieser Beschreibung daher als "LED-Stift" bezeichnet.

In der vorliegenden Beschreibung bezieht sich der Begriff "distal" auf von dem Nutzer, z.B. dem Chirurgen, entfernt gelegene Bereiche, während "proximal" sich auf dem Nutzer näherliegende Bereiche der Vorrichtung bezieht.

## Detaillierte Beschreibung der Erfindung

Die vorliegende Erfindung stellt eine Vorrichtung (1) zur gezielten Beleuchtung eines intraokularen Raumes eines menschlichen oder tierischen Auges bereit, die einen Sockel (2) mit einem proximalen Ende (23) und einem distalen Ende (22), einen Schaft (3) mit einem proximalen Ende und einem distalen Ende, eine Sonde (4) mit einem proximalen Ende und einem distalen Ende, eine Lichtquelle (5) mit mindestens einer LED, eine mit der Lichtquelle (5) verbundene Energiequelle (6) umfasst, wobei der Schaft an seinem distalen Ende mit dem proximalen Ende des Sockels verbindbar ist, die Sonde mit dem distalen Ende des Sockels verbindbar ist und die Lichtquelle (5) am distalen Ende der Sonde (4) positioniert ist und das distale Ende der Sonde bildet und nicht innerhalb der Sonde (4) oder des Schaftes (3) angeordnet ist, so dass das von der Lichtquelle (5) emittierte Licht direkt in das Auge eingestrahlt wird, ohne über einen Lichtleiter geführt zu werden, wobei der Abstrahlwinkel der mindestens einen LED durch einen Reflektor begrenzt oder durch eine Verkapselung der LED mit einem transparenten Material eingestellt ist. Bei der erfindungsgemäßen Vorrichtung handelt es sich somit um einen Ophthalmoilluminator, der insbesondere zur Ausleuchtung des Auges geeignet ist.

Die erfindungsgemäße Vorrichtung, d.h. der erfindungsgemäße Ophthalmoilluminator, zeichnet sich dadurch aus, dass er sehr flexibel einsetzbar ist, je nach Bedarf angepasst werden kann, das Operationsgebiet gezielt und optimal ausleuchten kann und gleichzeitig das restliche Auge, insbesondere die Netzhaut schützt. Der erfindungsgemäße Ophthalmoilluminator ist daher für die Ophthalmochirurgie und für die Analytik sehr gut geeignet, z.B. für das Einfärben und Entfernen von Membranen im Auge, oder für Eingriffe am Augenhintergrund, z.B. an der Netzhaut und für jede Art von Pars plana Vitrektomie. Weiterhin ist der erfindungsgemäße Ophthalmoilluminator auch für Differenzialdiagnose geeignet, z.B. für die Unterscheidung von sauerstoffreichem und sauerstoffarmem Gewebe oder von autofluoreszierendem Gewebe. Die erfindungsgemäße Vorrichtung zeichnet sich auch dadurch aus, dass sie kompakt ist, dass keine externen Verbindungen mit einer Lichtquelle erforderlich sind, die den Operateur beeinträchtigen könnten. Die Vorrichtung ist steril oder sterilisierbar und kann wiederholt oder als Wegwerfprodukt eingesetzt werden. Dadurch ist sie überall einsetzbar, auch unter schwierigen Bedingungen. Weiterhin wurde gezeigt, dass mit der erfindungsgemäßen Vorrichtung die in der obengenannten DIN-Norm vorgeschriebenen Grenzwerte eingehalten werden können und dass eine ausreichende Expositionszeit ermöglicht wird.

Der Sockel der erfindungsgemäßen Vorrichtung ist ein Teil, das dazu dient, am distalen Ende eine Sonde und am proximalen Ende einen Schaft zu halten, aufzunehmen oder abzustützen. Der Sockel kann eine Öffnung oder einen Kanal aufweisen, wobei die Öffnung bevorzugt rund bzw. im Wesentlichen rund ist und wobei der Kanal gerade oder gebogen sein kann und der Querschnitt des Kanals bevorzugt rund oder oval ist. Der äußere Teil des Sockels kann jegliche Form haben, bevorzugt ist er rund oder oval. Die äußere Wand kann z.B. gerade, konisch oder doppelkonisch ausgebildet sein. Die Öffnung des Sockels bzw. der Kanal dient dazu, eine oder mehrere Stromleitungen hindurchzuführen. Der Sockel kann eine Aufnahmestelle für den Schaft aufweisen, mit dem Schaft verbunden oder Teil des Schaftes sein. Der Sockel kann dazu dienen, eine Sonde hindurchzuführen oder aber an einem Ende eine Sonde aufzunehmen. Am anderen, dem proximalen Ende, ist ein Schaft anbringbar.

Der Schaft der erfindungsgemäßen Vorrichtung kann jede beliebige Form haben. Er dient dazu, die Vorrichtung zu halten und/oder zu navigieren. Der Schaft kann z.B. eine größere Dimensionierung aufweisen und in sich die gesamte Elektronik und Stromversorgung verstauen. Der Schaft kann z.B. integraler Teil eines Stiftes sein, welcher auf der Außenhülle die benötigten Bedienelemente für Strahlungsfluss, Farbton, Frequenz, etc. aufweist.

Die Sonde der erfindungsgemäßen Vorrichtung ist stabförmig, wobei der Querschnitt des Stabs jede beliebige Form haben kann. Die Sonde dient dazu die Lichtquelle zu halten oder zu tragen und ggf. eine Verbindung von Lichtquelle zu Energiequelle, z.B. eine Stromleitung aufzunehmen. Die Sonde kann starr oder flexibel sein, sie kann z.B. durch den Einsatz von Memory-Legierungen steuerbar ausgeprägt sein.

Eine wesentliche Eigenschaft des erfindungsgemäßen Ophthalmoilluminators ist es, dass der intraokulare Raum gezielt und optimal ausgeleuchtet werden kann. Dies wird zum einen dadurch erreicht, dass die Lichtquelle sich am distalen Ende der Vorrichtung befindet und nicht innerhalb der Sonde oder des Schaftes angeordnet ist, sondern das distale Ende der Sonde bildet, so dass Licht direkt in das Auge eingestrahlt wird, ohne über einen Lichtleiter geführt zu werden. Dadurch wird erreicht, dass wenige Verluste auftreten und die Effizienz hoch ist.

Außerdem kann das Licht direkt über die Bewegung der Sonde dahin gelenkt werden, wo es notwendig ist. Dies vermeidet auch die Notwendigkeit, Licht über eine Leitung von einer Lichtquelle herzuleiten, wozu relativ starre Leitungen notwendig sind, die während eines Eingriffs stören.

Ein weiteres wichtiges Merkmal ist es, dass der von der Lichtquelle emittierte Lichtstrahl vom Benutzer eingestellt bzw. geführt werden kann. Auf diese Weise kann genau der Bereich beleuchtet werden, den der Nutzer, z.B. der Operateur, sehen muss, während andere Bereiche der Netzhaut geschont werden können. Der Lichtstrahl bzw. Lichtkegel kann durch Bewegung des Schaftes navigiert werden. Größe, Form und Richtung des Lichtkegels können durch Auswahl und/oder Anpassung der LED und/oder der Sonde und durch Navigierung verändert werden.

Die beschriebenen Eigenschaften der erfindungsgemäßen Vorrichtung lassen es zu, die Vorrichtung für viele verschiedene Zwecke zu verwenden, da der Lichtstrahl genauer fokussiert und dosiert werden kann, als bei aus dem Stand der Technik bekannten Geräten. Außerdem wurde gefunden, dass der Glaskörper nicht nur für sichtbares Licht sondern auch für IR- und UV-Strahlung ausreichend transparent ist, sodass sowohl bei invasiver als auch bei nicht-invasiver transskleraler Verwendung ausreichend Strahlung am gewünschten Ort ankommt.

Eine Möglichkeit zur Variation einer LED ist die Einstellung des Abstrahlwinkels. Der Abstrahlwinkel einer LED kann mit der Formel

$$\alpha = 2 * \left( \arctan\left(\frac{d}{l * 2}\right)\right)$$

berechnet werden, wobei

$\alpha$ = Abstrahlwinkel
d = Durchmesser des zu beleuchtenden Objektes/Raumes
l = Abstand der Lichtquelle zur beleuchtenden Fläche
arctan = Umkehrfunktion der Tangensfunktion

Entsprechend kann eine geeignete LED für den jeweiligen Zweck ausgewählt werden. Der Abstrahlwinkel kann gemäß der Erfindung verändert werden, z.B. durch den Reflektor begrenzt werden. Der Lichtstrahl kann durch eine Linse gestreut werden, bzw. der Lichtkegel kann durch eine Linse aufgeweitet oder kollimiert werden. Die Richtung des Lichtstrahls/Lichtkegels kann verändert werden, indem der Bereich der Lichtquelle, der den Lichtkegel aussendet, entsprechend ausgebildet wird. Diese Anpassungen sind dem Fachmann bekannt. Der Abstrahlwinkel (Öffnungswinkel) $\alpha$ der Lichtquelle kann gemäß der Erfindung auch durch entsprechende Verkapselung der LED eingestellt werden.

Geeigneterweise wird die LED so ausgewählt, dass das Auge nicht oder kaum thermisch belastet wird.

Eine weitere Möglichkeit, den Lichteinfall zu beeinflussen, besteht darin, einen Teil der Oberfläche der LED so zu beschichten, dass kein oder wenig Licht an unerwünschten Stellen austritt, sodass die Hauptmenge an Licht über einen gewünschten Bereich austreten kann. Damit kann dann bei der Beleuchtung noch gezielter die gewünschte Stelle angestrahlt werden, wobei empfindlichere Stellen von der Beleuchtung dann ausgenommen werden.

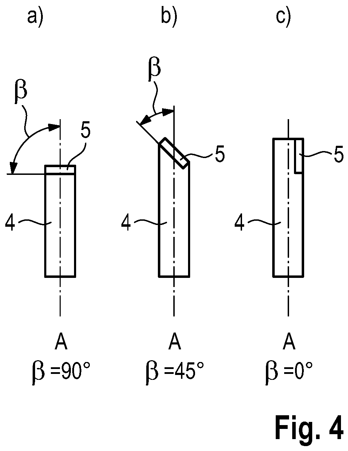

In einer weiteren Ausführungsform wird der Lichtkegel dadurch beeinflusst, dass die Lichtquelle in einem Winkel ß an der Sonde angebracht wird. Der Winkel ß bezieht sich dabei auf den Winkel, der zwischen der Längsachse (A) der Sonde und der Unterseite der Lichtquelle gebildet wird. So kann z.B. die Lichtquelle (5) in einem Winkel ß von 0° oder 5° bis 90°, bevorzugt von 10° bis 80°, bevorzugt von 20° bis 70°, bevorzugt von 30° bis 60°, bevorzugt von 40° bis 50°, zum Beispiel in einem Winkel von 45° oder 0°. Beispielhaft ist in Figur 4 eine Anordnung der Lichtquelle (5) an der Sonde (4) in einem Winkel ß von 90° (Figur 4a) , 45° (Figur 4b) bzw. 0° (Figur 4c) gezeigt.

Der von der Lichtquelle emittierte Lichtkegel kann über den Schaft der Vorrichtung manuell durch Drehen oder Kippen gezielt navigiert werden. Eine lokale Ausrichtung des Lichtkegels durch Drehen erfolgt insbesondere dann, wenn die Lichtquelle nicht direkt vorne auf der Sonde aufsitzt, sondern in einem Winkel dazu angebracht ist. Die lokale Ausrichtung kann auch durch Kippen der Vorrichtung erfolgen. Hierzu ist in einer bevorzugten Ausführungsform der Schaft so ausgebildet, dass er leicht vom Operateur bedient werden kann. Beispielsweise kann der Schaft Längsrippen aufweisen, um seine Griffigkeit zu erhöhen.

Die einfache Bedienbarkeit, d.h. Drehen oder Kippen, wird dadurch erzielt, dass die erfindungsgemäße Vorrichtung über den Sockel in einem Gewebe "verankert" oder gehalten werden kann. Mit anderen Worten kann der Sockel in Gewebe gesteckt werden, insbesondere Gewebe in der Pars plana. In dieser Ausführungsform kann der Sockel beispielsweise einfach oder doppelt konisch geformt sein, um das "Verankern" zu erleichtern. Unter Verankern wird hierbei verstanden, dass der Sockel in einen Gewebebereich geschoben wird und dort an Ort und Stelle verbleibt. Die Sonde der erfindungsgemäßen Vorrichtung kann auch in einen bereits in das Gewebe insertierten Trokar geführt werden, wobei der Trokar dann für die Verankerung sorgt.

Aufgrund der im Winkel ß angeordneten Lichtquelle kann ein Operationsgebiet an Stellen optimal und gewebeschonend ausgeleuchtet werden, die mit bekannten Geräten nicht erreicht werden, z.B. Bereiche in der Nähe der Insertionsstelle oder an der Peripherie der Retina. Andererseits kann durch einen am Sockel vorgesehenen Abstandshalter verhindert werden, dass die Sonde mit der LED zu nahe an die empfindliche Netzhaut kommt.

So ist es möglich, den Lichtkegel im Auge extrem zielgerichtet auf die zu beleuchtende Stelle zu richten. Auch kann gezielt die Beleuchtung empfindlicher Stellen, wie beispielsweise die Makula, von der Beleuchtung ausgenommen werden. Gleichzeitig wird durch die zielgerichtete Navigation und eingeschränkte Größe des Lichtkegels vermieden, dass weiteres Gewebe unnötig der Licht- und Wärmestrahlung ausgesetzt wird.

Die LED kann mit jedem für diese Art von Lichtquelle geeigneten Material, insbesondere transparentem Material verkapselt sein. Geeignet ist beispielsweise transparentes Glas, Keramik oder ein Polymer wie Epoxidharz, Acrylharz, Plexiglas, Polymethylmethacrylat, Polyurethan oder Silikon. Die Wahl des Materials bestimmt die optische Qualität, die in der Regel bei härteren Materialien wie Glas höher ist, und die Gewebeverträglichkeit, die eher bei weicheren Materialien wir Silikon höher ist. Es kann auch ein Material verwendet werden, das als Diffusor wirkt oder das Licht durch Beugung in gewünschter Weise verändert.

Die Lichtquelle kann eine plane, konkave oder konvexe Oberfläche mit einem runden, abgerundeten oder mehreckigen Querschnitt haben. Vorteilhafterweise sind die Kanten der Lichtquelle, bzw. der Verkapselung der Lichtquelle abgerundet bzw. abgekantet, um mechanische Reizung und Absplitterungen während der Verwendung des Ophthalmoilluminators am Auge zu vermeiden.

Sowohl Kapselmaterial als auch Kapselform können gezielt gewählt werden, um die Lichtführung, Abstrahlrichtung und Lichtqualität der Lichtquelle zu beeinflussen.

Die erfindungsgemäße Vorrichtung kann weiterhin eine Steuereinrichtung umfassen, über die die Farbtemperatur und/oder Wellenlänge und/oder Helligkeit und/oder Intensität des Lichts einstellbar ist. Die Steuereinrichtung kann in üblicher Art und Weise ausgebildet sein. Es kann sich z.B. um eine drahtlose Schnittstelle handeln, die über eine Fernbedienung gesteuert werden kann. Jede mögliche Art der Steuerung ist hier möglich, z.B. auch eine Steuerung über Sprache oder Bewegung.

Um die Farbtemperatur des Lichts einzustellen, kann entweder gezielt eine LED in der gewünschten Farbtemperatur oder eine Kombination von zwei oder mehr LEDs gewählt werden, die dann die gewünschte Farbe bilden können. Ein Beispiel ist eine als RGB LED bezeichnete Lichtquelle, die aus drei hintereinandergeschalteten LEDs besteht, aus denen sehr viele gewünschte Farben gemischt werden können. Eine Veränderung der Farbtemperatur kann vorteilhaft sein, z.B. um spezifische Gewebestrukturen unter speziellem Licht besser erkennen zu können, um Operationsgeräte unter speziellem Licht besser erkennen und einsetzen zu können, um spezifisch für die Färbung von Strukturen eingesetzte Farbstoffe besser erkennen zu können, oder um die Farbtemperatur speziell auf einen Nutzer einstellen zu können. So kann z.B. die Wellenlänge vor und/oder während einer Operation auf verwendete Farbstoffe, Farbpigmente bzw. nanoskalige fluoreszierende Partikel oder Flüssigkeiten abgestimmt werden.

Weiterhin kann die Helligkeit der Lichtquelle über eine gezielte Schaltung mit verschiedenen Strömen eingestellt werden.

Die Steuereinrichtung, über die die Farbtemperatur des Lichts und/oder die Helligkeit des Lichts einstellbar ist, kann als Schalter mit verschiedenen Schaltstufen ausgeführt sein. In einer anderen Ausführungsform erfolgt die Schaltung elektronisch, z.B. über eine Sprachsteuerungseinheit oder eine Fernbedienung. Diese kann extern oder in den Schaft integriert vorliegen.

Die Einstellung der Parameter Austrittswinkel des Lichts, Abstrahlwinkel des Lichts, Farbtemperatur und Helligkeit ermöglichen die bestmögliche Ausleuchtung des Operationsgebietes im Auge bei gleichzeitiger bestmöglicher Schonung des betroffenen und des angrenzenden Gewebes, sowie bestmöglichem Makulaschutz.

Aus den verschiedenen Teilen der erfindungsgemäßen Vorrichtung kann flexibel eine für den jeweiligen Zweck besonders gut geeignete Vorrichtung gestaltet werden. Wesentliche Teile hierzu sind die Sonde, der Sockel, der Schaft und die Lichtquelle. Alle Teile der Vorrichtung können aus an sich für diese Anwendung üblichen Materialien hergestellt werden. Das jeweilige Material sollte biokompatibel und sterilisierbar sein.

Der Sockel dient dazu, die Vorrichtung bei intraokularer Verwendung im Gewebe zu halten und/oder zu verankern und die Sonde auf der distalen Seite und den Schaft auf der proximalen Seite zu halten. Der Sockel ist hierzu als Teil mit einer Öffnung ausgebildet. Der Sockel kann aus üblichen biokompatiblen und bevorzugt auch elektrisch isolierenden Materialien hergestellt sein. Beispielsweise kann der Sockel aus gewebeverträglichem elastomeren Material, z.B. elastomerem Silicon, elastomeren Polymeren etc. hergestellt sein. Durch eine gewisse Elastizität kann sich der Sockel dann der Umgebung anpassen. Allerdings darf das Material nicht zu flexibel sein, da der Durchgang, d.h. die Öffnung, zugänglich sein sollte. Bei geeigneter Formgebung ist ebenfalls auch ein Duroplast als Material für den Sockel geeignet. Darüber hinaus kann der Sockel dazu dienen, die Vorrichtung an einer gewünschten Stelle im Gewebe, z.B. im Pars plana Bereich und im geeigneten Abstand zu halten.

Auf der distalen Seite des Sockels kann eine Sonde positioniert werden. Die Sonde dient dazu, an ihrer Vorderseite die Lichtquelle zu tragen und in ihrem Inneren die Verbindung von der Lichtquelle zur Energiequelle zuzulassen. Hierzu werden in der Regel Verbindungen, z.B. Drähte oder ein leitfähiges Polymer, verwendet, die die Lichtquelle mit der proximal des Sockels gelegenen Energiequelle verbinden. Die Sonde soll möglichst dünn sein, um möglichst wenig Gewebe zu schädigen. Das Material der Sonde sollte elektrisch isolierend sein.

Bevorzugt liegt die Dicke der Sonde im Bereich von 20 bis 30 gauge, besonders bevorzugt 23 bis 27 oder 28 gauge.

Ein weiterer wesentlicher Teil der erfindungsgemäßen Vorrichtung ist ein Schaft, der an der proximalen Seite des Sockels positioniert ist. Der Schaft kann entweder fest mit dem Sockel verbunden sein oder aber der Sockel kann so ausgebildet sein, dass er den Schaft aufnehmen und halten kann. Der Schaft dient dazu, die Vorrichtung zu bedienen, d.h. zu positionieren, zu drehen und zu kippen. In einer Ausführungsform ist der Schaft daher so ausgebildet, dass er Griffmulden, Griffrippen, Griffflächen oder Ähnliches aufweist, damit er leicht vom Nutzer gedreht und gehalten werden kann. Der Schaft kann aus jedem möglichen Material hergestellt sein, solange das Material biokompatibel ist. Bevorzugt wird für den Schaft sterilisierbares und/oder isolierendes Material verwendet. Die Sterilisierung kann z.B. mit Gas und/oder Gammastrahlen erfolgen.

Die erfindungsgemäße Vorrichtung kann in einer Ausführungsform zum leichteren Einsetzen zusätzlich zu dem Schaft noch einen Handgriff aufweisen, der abnehmbar an dem Schaft oder an dem Sockel positionierbar ist. Nach dem Einsetzen der Vorrichtung kann der Handgriff abgenommen werden. Es ist auch möglich, dass an dem Sockel zuerst ein Handgriff positioniert ist zum Einsetzen der Vorrichtung und nach dem Einsetzen der Handgriff dann durch den Schaft ausgetauscht wird. Der Handgriff ist in der Regel größer als der Schaft und könnte während eines Eingriffs behindern, so dass es vorteilhaft ist, ihn nach dem Einsetzen zu entfernen.

Die Lichtquelle (5) ist mit einer Energiequelle elektrisch verbunden, wobei die Lichtquelle distal an einer Sonde positioniert ist, während die Energiequelle bevorzugt außerhalb des intraokularen Raums an der Vorrichtung angeordnet ist, z.B. im proximalen Bereich des Schaftes. Die Verbindung von Lichtquelle und Energiequelle kann kontaktlos oder über elektrische Leitungen erfolgen. In einer Ausführungsform ist die Lichtquelle über einen Schalter mit einer Energiequelle (6) verbunden. Die Energiequelle (6) kann eine oder mehrere Batterien, einen oder mehrere Akkus, einen oder mehrere Kondensatoren oder Kombinationen davon umfassen. In einer bevorzugten Ausführungsform weist die Energiequelle mindestens eine Batterie auf. Geeignet sind Batterien wie sie für medizintechnische Geräte verwendet werden. Bevorzugt werden Lithiumionen-Batterien oder Batterien bzw. Stromquellen mit vergleichbaren Eigenschaften verwendet, da diese einen sehr kontinuierlichen Strom liefern und somit die Leuchtintensität der Lichtquelle ziemlich konstant bleibt. Eine konstante Lichtintensität trägt zu den vorteilhaften Eigenschaften des erfindungsgemäßen Ophthalmoilluminators bei.

Um die Stabilität des ausgestrahlten Lichts weiter zu verbessern und die Lichtintensität konstant zu halten, kann in einer weiteren bevorzugten Ausführungsform zusätzlich ein Strombegrenzer oder LED-Treiber verwendet werden.

Bevorzugt befindet sich die Energiequelle in einem dafür vorgesehenen Gehäuse. Das Gehäuse wird vorzugsweise nahe des proximalen Bereichs des Schaftes fixiert oder in den Schaft integriert, um eine möglichst kompakte Vorrichtung zur Verfügung zu stellen. Möglich ist auch die Verwendung von mindestens einem Akku oder Kondensator, der berührungslos oder durch Verbindung mit einem Ladegerät geladen werden kann. So kann das Gehäuse, das die Energiequelle enthält und mit der Lichtquelle verbunden ist, während eines Eingriffs beispielsweise in der Nähe der Vorrichtung, z.B. auf die Stirn des Patienten, aufgeklebt werden.

Die Verwendung von Batterien oder Akkus als Energiequelle ermöglicht den vielseitigen Einsatz des erfindungsgemäßen Ophthalmoilluminators.

In einer Ausführungsform der erfindungsgemäßen Vorrichtung ist die Sonde an dem Sockel befestigt und wird über eine Halterung und/oder den Schaft durch das Gewebe geführt, z.B. im Pars plana Bereich. In dieser Ausführungsform wird zuerst eine Öffnung im Gewebe erzeugt, z.B. durch eine Inzision, durch die dann die Sonde mit dem Sockel geschoben werden kann.

In einer weiteren Ausführungsform wird zuerst mit Hilfe eines üblichen Punktionsinstruments, z.B. ein Trokar, in das Gewebe gestochen. Anschließend wird dann die erfindungsgemäße Sonde durch den Trokar, in den Intraokularraum geschoben. Für diese Ausführungsform muss die Sonde mit Lichtquelle einen kleineren Durchmesser haben als der Trokar, um durchgeschoben werden zu können. In dieser Ausführungsform ist bevorzugt die Sonde (4) mit reduziertem Außendurchmesser ausgestaltet, z.B. 18 bis 30 gauge, bevorzugt 23 bis 27 oder 28 gauge.

Die oben beschriebenen Ausführungsformen haben viele Vorteile gegenüber bekannten Vorrichtungen: sind einfach in der Anwendung, sehr flexibel und können an viele verschiedene Gegebenheiten angepasst werden. Sie können in den Intraokularraum mit minimal invasiver Technik eingeführt werden, liefern eine optimale Ausleuchtung, die außerdem noch während des Eingriffs bzgl. verschiedener Parameter angepasst werden kann.

In einer Ausführungsform des erfindungsgemäßen Ophthalmoilluminators befinden sich alle Teile, d.h. Sockel, Schaft, Sonde, Lichtquelle, Energiequelle und Gehäuse bereits fertig montiert kompakt in einem Gehäuse, d.h. alle Bestandteile bilden eine Einheit, sind integriert und können als integrale Vorrichtung bezeichnet werden. Diese Ausführungsform ist geeignet für transsklerale Illumination. Die erfindungsgemäße Vorrichtung kann dabei ausgebildet sein wie ein Stift, an dessen Spitze sich eine Lichtquelle befindet. Das Gehäuse kann über die gesamte Länge im Wesentlichen den gleichen Außendurchmesser aufweisen. Diese Ausführungsform kann insbesondere dazu verwendet werden, um den intraokularen Raum transskleral zu beleuchten. Dazu kann die Vorrichtung in der Bindehauttasche geführt werden und den intraokularen Raum über die Sklera beleuchten. Der Stift wird mit der Hand geführt, d.h. der Nutzer kann das Licht genau dahin lenken, wo er es braucht. Das Licht fällt durch die Sklera. Dieser Stift dient für Diagnostik, zur Beleuchtung und gleichzeitig als Deller. Bei dieser Ausführungsform wird die Netzhaut von außen beleuchtet und Risse und Löcher werden für den Nutzer gut sichtbar. Diese Ausführungsform ist daher gut geeignet für die nicht-invasive Untersuchung. Ein weiterer Vorteil dieser Vorrichtung ist es, dass keine nach außen gehenden Drähte, Verbindungen oder Halterungen notwendig sind. Der Stift kann als Wegwerfprodukt hergestellt werden. Er wird der Verpackung entnommen, ist steril, wird verwendet und dann weggeworfen. Er ist daher gut geeignet für den Einsatz im Feld, in schwierigen Gebieten, im Lazarett usw.

In einer Ausführungsform dieser stiftförmigen Vorrichtung können mehrere LEDs, z.B. RGB-LEDs verwendet werden, da hier genug Platz dafür ist. Dann kann die Farbe des Lichtes jeweils vom Nutzer nach Wunsch eingestellt werden.

In einer anderen Ausführungsform ist ein proximaler Bereich (11) des Schaftes als Handstück (42) zur Navigation des Lichtkegels ausgestaltet. Das Handstück weist bevorzugt einen größeren Außendurchmesser als der Rest des Schaftes auf. In einer weiteren Ausführungsform ist die Oberfläche des Handstückes und/oder des Schaftes modifiziert, um eine bessere Griffigkeit zur Navigation des Schaftes und damit des Lichtkegels zu erreichen. Alternativ kann ein kleiner Griff in Form eines senkrecht zum Schaft angebrachten Stabes oder einer anderweitigen Ausstülpung am Handstück angebracht werden, um die Feinnavigation zu erleichtern.

Ferner ist in einer weiteren Ausführungsform der erfindungsgemäßen Vorrichtung im proximalen Bereich des Schaftes (11) eine Halterung (42) angebracht. Diese Halterung dient der besseren Handhabbarkeit z.B. bei der Insertion des erfindungsgemäßen Ophthalmoilluminators in eine am Pars plana eingebrachte Insertion. Die Halterung ist so am Schaft angebracht, dass sie de-arretiert und entfernt werden kann, sobald diese nicht mehr benötigt wird. Es bleibt dann der Schaft oder das Handstück aus der alternativen Ausführungsform zurück und ermöglicht eine Feinnavigation des Schaftes, bzw. des Lichtkegels.

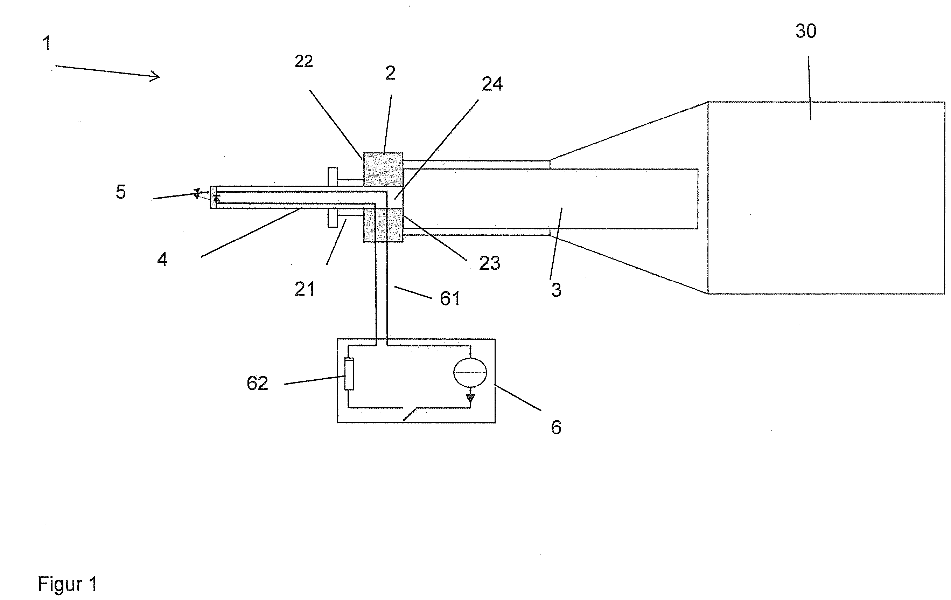

Fig. 1 zeigt eine Ausführungsform der erfindungsgemäßen Vorrichtung zur Beleuchtung eines intraokularen Raumes eines menschlichen oder tierischen Auges. Sockel 2 trägt am distalen Ende 22 eine Sonde 4. An dem Sockel befindet sich außerdem ein Abstandshalter 21, der dafür sorgt, dass die Sonde nicht zu weit in den intraokularen Raum hineingeschoben werden kann, um dadurch eine zu starke Strahlenbelastung bzw. Verletzungen durch die Sonde zu verhindern. Am distalen Ende der Sonde 4 befindet sich als Lichtquelle eine LED 5. Die Lichtquelle ist mit einer außerhalb der Vorrichtung angeordneten Energiequelle 6 über eine Leitung 61 verbunden. Die Leitung führt von der LED 6 durch die Sonde 4 und einen Kanal 24 in dem Sockel 2 zu dem Batteriegehäuse 6, in dem sich ggf. eine oder mehrere Batterien (nicht gezeigt) als Energiequelle befinden. Das Batteriegehäuse 6 weist außerdem einen Strombegrenzer 62 auf. An der proximalen Seite 23 des Sockels 2 befindet sich ein Schaft 3, der zur Navigation des Lichtkegels vorgesehen ist, wenn die Vorrichtung zur Beleuchtung in Betrieb genommen wird. Auf den Schaft 3 ist eine Halterung 30 aufgesteckt, die zur Applikation der Vorrichtung dient und, sobald die Vorrichtung im Gewebe verankert ist, abgenommen werden kann.

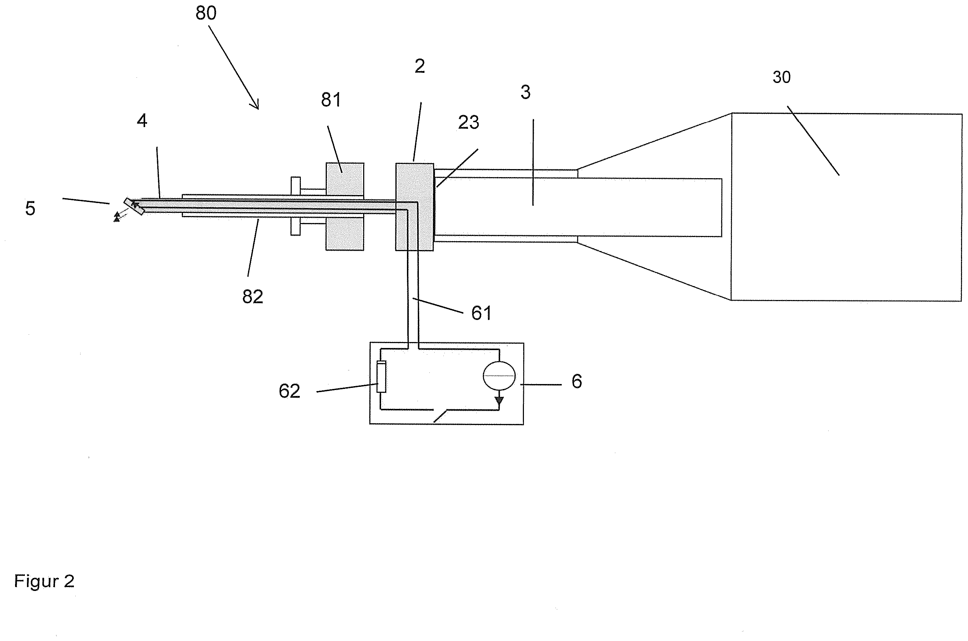

Eine weitere Ausführungsform der erfindungsgemäßen Vorrichtung ist in Fig. 2 dargestellt. Die in Fig. 2 dargestellte Vorrichtung zur gezielten Beleuchtung eines intraokularen Raumes eines menschlichen oder tierischen Auges weist einen Sockel 2 auf, der am proximalen Ende 23 einen Schaft 3 trägt, der zur Navigation des Lichtkegels geeignet ist. Auf den Schaft 3 aufgesteckt ist eine Halterung 30, die zur Applikation der Vorrichtung dient und abgenommen werden kann, sobald die Vorrichtung im Gewebe verankert ist. Am distalen Ende 21 trägt der Sockel 2 eine Sonde 4, an deren distalem Ende eine LED 5 so positioniert ist, dass sie mit der Achse der Vorrichtung einen Winkel ß von 45° bildet. Von der LED 5 führen Drähte 61 durch einen Kanal (nicht gezeigt) in der Sonde 4 hindurch in den Sockel 2 und über einen im Winkel angeordneten im Sockel vorgesehenen Kanal aus der Vorrichtung hinaus hin zu einem Batteriegehäuse 6, das eine oder mehrere Batterien (nicht gezeigt) aufweist. Dadurch ist die LED 5 mit einer Stromquelle, dem Batteriegehäuse 6 verbunden. Das Batteriegehäuse 6 weist außerdem einen Strombegrenzer 62 auf. Die Sonde 4 führt in der Ausführungsform der Fig. 2 durch einen Trokar 80 hindurch, der im Gewebe verankert ist. Der Trokar 80 besteht aus einem Halteteil 81 und einer Kanüle 82. Die Kanüle 82 dient dazu, das Gewebe zu durchstechen und die Sonde des erfindungsgemäßen Ophthalmoilluminators zu führen und zu halten. Bei Verwendung wird die Sonde 4 der erfindungsgemäßen Vorrichtung durch den in dem Trokar 80 und die Kanüle 82 gebildeten Kanal in den Intraokularraum geführt, um intraokular zu beleuchten. Wenn die Beleuchtung nicht mehr notwendig ist, kann die Vorrichtung wieder herausgezogen werden und andere Instrumente können durch den Kanal 82 des Trokars 80 eingeführt werden. Sobald wieder Beleuchtung notwendig ist, kann wiederum die Sonde eingeführt werden. Diese Ausführungsform ist sehr schonend und flexibel, da ein insertierter Trokar für verschiedene Zwecke genutzt werden kann, der Trokar als Anschlag dient, damit die Sonde nicht zu weit in den Okularraum geführt werden kann, und nur eine Inzision im Gewebe notwendig ist für Beleuchtung und Instrumente. Diese vorteilhafte Ausführungsform ermöglicht es, bei der Ophthalmoillumination am Pars plana, zunächst nur den Instrumentenkanal in die Inzision am Auge einzubringen und dann im zweiten Schritt den Ophthalmoilluminator durch den Instrumentenkanal schonend ins Auge einzubringen und auch frei zu drehen, ohne weitere Reibung an der Sklera.

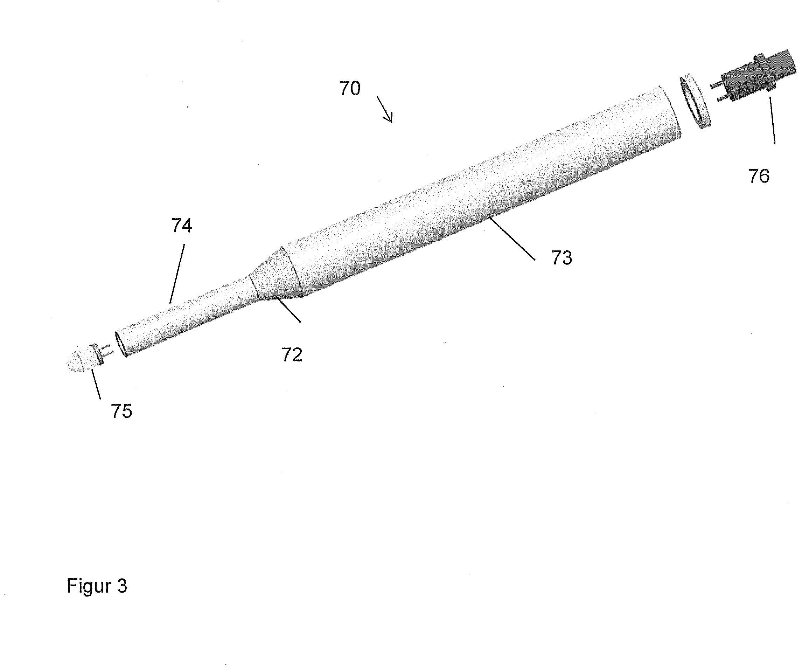

Fig. 3 zeigt eine weitere Ausführungsform der erfindungsgemäßen Vorrichtung, die alle erfindungswesentlichen Komponenten in einem stiftförmigen Gehäuse enthält - einen LED-Stift 70. Diese sehr kompakte Ausführungsform, die keinerlei externe Quellen für Energie oder Licht benötigt, ist überall einsatzbereit. Fig. 3 zeigt das Gehäuse 70 mit einer Sonde 74, an deren distalem Ende eine Lichtquelle 75 (LED) aufsteckbar ist. Ein konusförmiger Sockel 72 befindet sich zwischen Sonde 74 und Schaft 73. Am proximalen Ende des Schafts 73 ist ein Schalter aufsteckbar, mit dem die Lichtquelle an- und ausgeschaltet werden kann. In dem Gehäuse befindet sich eine Energiequelle (nicht gezeigt), die über eine Leitung mit der Lichtquelle verbunden ist (ebenfalls nicht gezeigt). Die LED des Stifts kann in der Bindehauttasche geführt werden und beleuchtet die Netzhaut von hinten, sodass der Benutzer Risse und Löcher gut sehen kann.

Alle Ausführungsformen wurden im Labor getestet und erfüllten die geforderten Grenzwerte und erlaubten eine Ausleuchtung des intraokularen Raums im gewünschten Umfang.

Die erfindungsgemäße Vorrichtung eignet sich zur Verwendung in chirurgischen und diagnostischen Verfahren am Auge, insbesondere zur minimalinvasiven, intraokularen Beleuchtung des intraokularen Raumes des menschlichen oder tierischen Auges.

Die erfindungsgemäße Vorrichtung eignet sich zur Verwendung in chirurgischen und diagnostischen Verfahren am Auge, sowohl für invasive als auch nicht-invasive Verfahren, z.B. eignet sie sich zur nicht-invasiven, transskleralen Beleuchtung des intraokularen Raumes des menschlichen oder tierischen Auges.

Für die nicht-invasive Anwendung sind insbesondere die Ausführungsformen der Vorrichtung geeignet, welche eine Lichtquelle mit einer abgerundeten Spitze haben. Diese kann dann auch verwendet werden, um bei der transskleralen Illumination gezielten Druck auf die Netzhaut auszuüben, um einerseits die Sicht zu verbessern und andererseits die Netzhaut beispielsweise bei einer Laserbehandlung zu fixieren.

Aufgrund der vielen Möglichkeiten zur Einstellung und Navigation der LEDs, zur Fokussierung und Dosierung des Lichts und zur Kombination von Wellenlängen ist die erfindungsgemäße Vorrichtung vielfach einsetzbar. Durch den Aufbau der Vorrichtung und die Positionierung der Lichtquelle kann gezielt Licht an eine ausgewählte Stelle gestrahlt werden und die Lichtstärke und Dauer begrenzt werden. Dadurch ist es beispielsweise möglich UV-Licht zur Detektion von fluoreszierenden Verbindungen im okularen Raum einzusetzen, ohne das Auge zu schädigen. Die Detektion fluoreszierender Verbindungen kann z.B. von Vorteil sein, um autofluoreszierende Gewebe oder Fluoreszenzmarker nachzuweisen.

Für die Diagnostik im okularen Raum kann die erfindungsgemäße Vorrichtung vorteilhaft eingesetzt werden, z.B. in der Tumordiagnostik. Tumorgewebe unterscheidet sich im Sauerstoffverbrauch und in der Sauerstoffsättigung von "normalem" Gewebe. Sauerstoffreiches Gewebe kann durch IR-Strahlung von sauerstoffarmem Gewebe differenziert werden. Da die erfindungsgemäße Vorrichtung mit Infrarot-LEDs betrieben werden kann, ist es möglich, durch transklerale Einstrahlung nicht-invasiv den okularen Raum sehr schonend auf sauerstoffreiches Gewebe zu untersuchen und damit zur Detektion von Tumorgewebe und der Neubildung von Tumorgewebe beizutragen.

Vorteil aller erfindungsgemäßen Vorrichtungen ist es, dass keine Verbindung zu einem stationären Gerät notwendig ist, insbesondere keine Verbindung von lichtleitenden Fasern zu einer Lichtquelle. Die Vorrichtung kann als Stift ausgeführt sein, welcher vorzugsweise als steriles Einmalprodukt ausgestaltet ist. Die Vorrichtung kann daher überall infrastrukturunabhängig verwendet werden und ist einfach in der Handhabung.

Die Erfindung wird auch durch die folgenden Beispiele, Vorrichtungen und Verwendungen und Verfahren, sowie Bestandteile derselben beschrieben und erläutert, ohne sie darauf zu beschränken.

**Beispiele**

**Beispiel 1: Stromversorgung durch ein Batterie-Reservoir**

- Die Stromversorgung wird durch ein Batterie-Reservoir ermöglicht. Diese kann mit mehreren Funktionen ausgestattet werden:

  ◦ Einfacher Schalter zur Inbetriebnahme des Illuminator
  ◦ Schaltung mit festen verschiedenen Strömen z.B.:

    ▪ I = 5 [mA]
    ▪ I = 10 [mA]
    ▪ I = 15 [mA]
    ▪ I = 20 [mA]
    ▪ Stufenlose oder Steuerung mit Stufen durch Mikrokontroller

  ◦ Steuerung durch Fernbedienung

**Beispiel 2: Leistungsdaten einer geeigneten LED:**

Eine für den Einsatz in der erfindungsgemäßen Vorrichtung geeignete LED hat folgende technische Daten:

| | |
|---|---|
| Leuchtfarbe: | Weiß |
| Farbkoordinaten: | x=0,31 y=0,31 |
| Lichtleistung: | typ. 98 mcd |
| Farbtemperatur: | typ. 5500 K |
| Spannung: | typ. 2,9 Volt |
| Strom: | max. 10 mA |
| Abstrahlwinkel: | 135° |
| Arbeitstemperatur: | - 40°C bis + 85°C |
| Löttemperatur: | 5 Sekunden 260°C |
| Maße: | 0,65 x 0,35 x 0,2 mm |

**Beispiel 3: Konstruktionsdaten einer geeigneten LED**

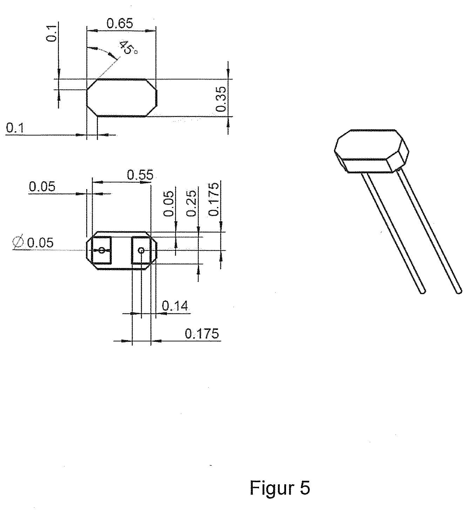

Figur 5 zeigt ein Beispiel für eine für den Einsatz in der erfindungsgemäßen Vorrichtung geeignete LED. Die in Figur 4 dargestellte LED hat eine plane Oberfläche mit einem mehreckigen, hier achteckigen Querschnitt und abgetrennte oder abgerundete Kanten, um eine Absplitterung der Kanten während der Verwendung am Auge zu vermeiden.

**Beispiel 4: Bestrahlungsstärke der LED:**

Die Bestrahlungsstärke der gesamten LED wurde mittels Ulbrichtkugel und gemittelten Werten einer Kalibrationslampe bei angegebenen maximalen Strom von I = 10 [mA] bestimmt.
Tabelle 1 stellt die so ermittelten Bestrahlungsstärken dar, die bei den Abständen von 0-18 mm gemessen wurden:

**Tabelle 1:**

| Distance [s] mm | Irradiation [$E_{300-850nm}$] $\mu$W cm-2 | Light flux [$\phi_V$] mim | Photochemical hazard | | Thermal hazard |
|---|---|---|---|---|---|
| | | | Irradiation [$E_{A-R}$] $\mu$W cm$^{-2}$ | Max. exposure time [$t_{max}$] h | Irradiation [$E_{VIS-R}$] $\mu$W cm$^{-2}$ |
| 0 | 1982.93 | 584.94 | 591.98 | 4.7 | 1972.24 |
| 1 | 385.07 | 107.76 | 127.51 | 21.8 | 383.10 |
| 2 | 163.14 | 47.80 | 49.50 | 57.9 | 162.25 |
| 3 | 76.30 | 22.31 | 23.26 | 120.8 | 75.89 |
| 4 | 44.79 | 13.02 | 13.78 | 204.6 | 44.55 |
| 5 | 26.51 | 7.68 | 8.22 | 338.5 | 26.36 |
| 6 | 16.89 | 4.88 | 5.23 | 533.0 | 16.80 |
| 7 | 12.21 | 3.51 | 3.83 | 727.5 | 12.14 |
| 8 | 9.37 | 2.70 | 2.93 | 949.4 | 9.33 |
| 9 | 7.46 | 2.11 | 2.43 | 1147.0 | 7.42 |
| 10 | 5.99 | 1.69 | 1.96 | 1425.0 | 5.95 |
| 11 | 4.80 | 1.35 | 1.57 | 1787.1 | 4.77 |
| 12 | 4.06 | 1.15 | 1.29 | 2156.1 | 4.04 |
| 13 | 3.44 | 0.97 | 1.13 | 2466.2 | 3.43 |
| 14 | 2.91 | 0.82 | 0.95 | 2959.0 | 2.90 |
| 15 | 2.56 | 0.72 | 0.86 | 3232.2 | 2.54 |
| 16 | 2.21 | 0.62 | 0.71 | 3903.4 | 2.20 |
| 17 | 1.92 | 0.54 | 0.61 | 4608.5 | 1.92 |
| 18 | 1.71 | 0.48 | 0.53 | 5232.7 | 1.71 |

(fortgesetzt)

| Distance [s] mm | Irradiation [$E_{300-850nm}$] μW cm-2 | Light flux [$\phi_V$] mim | Photochemical hazard | | Thermal hazard |
|---|---|---|---|---|---|
| | | | Irradiation [$E_{A-R}$] μW cm$^{-2}$ | Max. exposure time [$t_{max}$] h | Irradiation [$E_{VIS-R}$] μW cm$^{-2}$ |
| Limit for endoilluminators | | | 220.00 | | 350 000.00 |

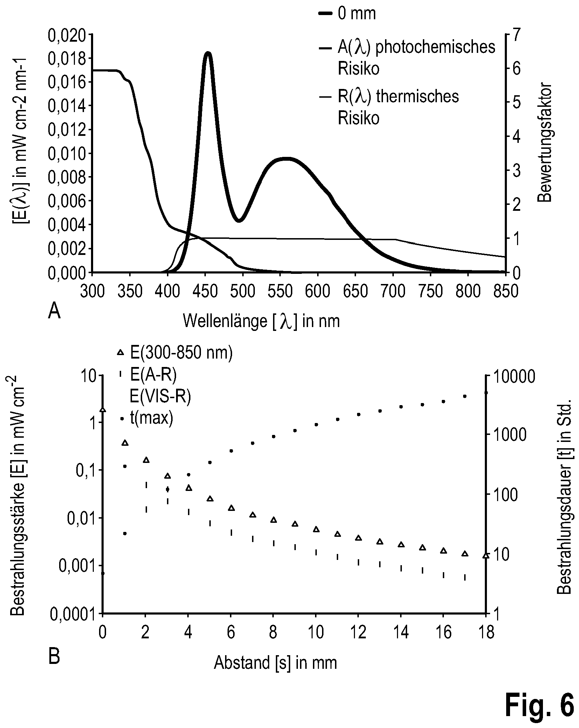

Figur 6A stellt die bei 10 mAStrömen gemessenen Bestrahlungsstärken in Abhängigkeit von der Wellenlänge des emittierten Lichtes bei 0 mm Abstand zur Detektionsfläche dar. In Figur 6B ist der Verlauf der Bestrahlungsstärke über den steigenden Abstand zur Bestrahlungsfläche zu sehen. Neben der Bestrahlungsstärke (300 -850 nm) in sichtbaren Spektralbereich auf einer Fläche von 1 mm, ist auf der Verlauf der gewicheten Bestrahlungsstärke laut "Ophthalmische Instrumente - Grundlegende Anforderungen und Prüfverfahren - Teil 2: Schutz gegen Gefährdung durch Licht (ISO/DIS 15004-2:2014); Deutsche Fassung prEN ISO 15004-2:2014" zu sehen.

**Beispiel 5: Bewertung nach ISO EN DIN15000-2.2104:**

Die LED wurde auf ihre photochemische Gefährdung hin untersucht und bewertet. Bestimmt wurden die gemessenen Werte mittels Ulbrichtkugel und gemittelten Werten einer Kalibrationslampe. Die gemessenen Werte wurden mittels einer 1 mm Blende erstellt, die restlichen Bewertungen wurden rechnerisch bestimmt.
Tabelle 1 stellt die so ermittelten Werte in Abhängigkeit des Abstands zwischen Emitter und Detektor dar. Der Abstand 0 mm stellt den Wert des "worst-case"-Falles dar. Dies wäre der Fall wenn die Lichtquelle die Netzhaut berühren würde. Es ergeben sich hier mögliche Zeiten von zwischen 4,7 h und 5232,7 h, welche wiederum realistisch für eine erfolgreich klinische Anwendung sind. Die ermittelten Werte zeigen, dass mit der hier gewählten LED bei der Verwendung in der erfindungsgemäßen Vorrichtung keine Gefährdung des Augengewebes zu erwarten ist. Die Tabelle 1 zeigt, dass für alle gemessenen Proben die Grenzwerte bzgl. der photochemischen Gefährdung eingehalten wurden, selbst wenn kein Abstand zur Netzhaut mehr da ist, d.h. bei Kontakt mit der Netzhaut. Allerdings soll bei der Verwendung der Vorrichtung für Untersuchungen am Auge der Kontakt mit der Netzhaut vermieden werden. Die Bestimmung der maximalen Emissionzeit $t_{max}$ durch die ermittelte Bestrahlungsstärke $E_{A-R}$ und der zulässigen gesamten Energiemenge pro Fläche von 10 J/cm$^2$, nach DIN EN ISO 15004-2:2014 zeigt, dass die Lichtquelle für den Einsatz im intraokularen Raum geeignet ist.
Die erhaltenen Werte zeigen, dass für die erfindungsgemäßen Vorrichtungen Expositionszeiten von weit mehr als 30 Minuten möglich sind. Dies lässt ihren breiten Einsatz in der Ophthalmochirurgie und -diagnostik zu.

**Beispiel 6: Test der Mikro-LED-Illumination an einem porkinen Auge**

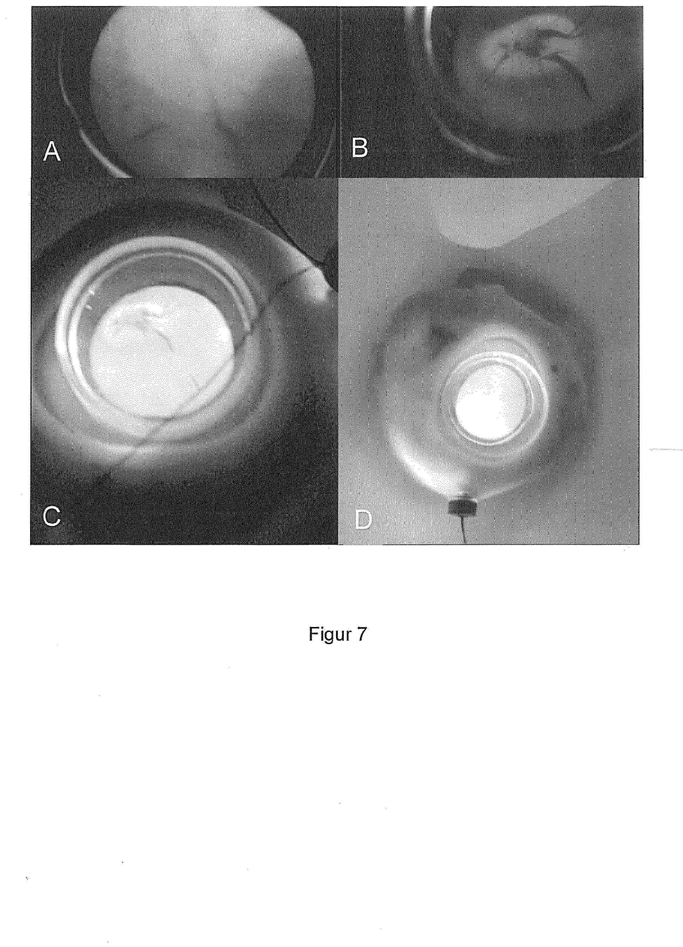

Figur 7 zeigt Bilder eines Beleuchtungstests mit einem erfindungsgemäßen Ophthalmoilluminator an einem porkinen Auge. A und B zeigen Bilder die mit einer Okularkamera mit sehr niedriger Belichtungszeit mit einem Zeiss-Operationsmikroskop aufgenommen wurden. C und D wurden mit einer Handykamera aufgenommen und spiegeln sehr gut das Helligkeitsempfinden wider. In D zeigte der Kegel der LED nicht in Richtung N. opticus, wie an der hellen Stelle an der Seite des Auges zu erkennen ist.

**Patentansprüche**

1. Vorrichtung (1) zur gezielten Beleuchtung eines intraokularen Raumes eines menschlichen oder tierischen Auges umfassend

    a) einen Sockel (2) mit einem proximalen Ende und einem distalen Ende,
    b) einen Schaft (3), mit einem proximalen Ende und einem distalen Ende,
    c) eine Sonde (4), mit einem proximalen Ende und einem distalen Ende,
    d) eine Lichtquelle (5) mit mindestens einer LED, und
    e) eine mit der Lichtquelle (5) verbundene Energiequelle (6),
    wobei der Schaft an seinem distalen Ende mit dem proximalen Ende des Sockels verbindbar ist, die Sonde am proximalen Ende mit dem distalen Ende des Sockels verbindbar ist und wobei die Lichtquelle (5) am distalen Ende der Sonde (4) positioniert ist und das distale Ende der Sonde (4) bildet und nicht innerhalb der Sonde (4) oder des Schaftes (3) angeordnet ist, so dass das von der Lichtquelle (5) emittierte Licht direkt in das Auge eingestrahlt wird, ohne über einen Lichtleiter geführt zu werden,

**EP 3 614 903 B1**

**dadurch gekennzeichnet, dass** der Abstrahlwinkel der mindestens einen LED durch einen Reflektor begrenzt oder durch eine Verkapselung der LED, insbesondere mit einem transparenten Material, eingestellt ist.

2. Vorrichtung (1) nach Anspruch 1, **dadurch gekennzeichnet, dass** die Lichtquelle (5) eine Unterseite aufweist, welche so angeordnet ist, dass zwischen Unterseite der Lichtquelle und einer Längsachse (A) des Schaftes ein Winkel β von 0° bis 90" gebildet wird.

3. Vorrichtung (1) nach einem der Ansprüche 1 oder 2, **dadurch gekennzeichnet, dass** Sockel und Sonde integral sind.

4. Vorrichtung (1) nach einem der vorhergehenden Ansprüche, **dadurch gekennzeichnet, dass** die Vorrichtung (1) eine Schnittstelle aufweist, die mit einer Steuereinrichtung verbindbar ist, über die Wellenlänge, Farbtemperatur, Intensität und /oder Helligkeit des Lichts einstellbar sind.

5. Vorrichtung (1) nach einem der vorhergehenden Ansprüche, **dadurch gekennzeichnet, dass** die Lichtquelle mindestens eine LED umfasst, die Licht mit einer Wellenlänge im nahen, mittleren oder fernen Infrarot abstrahlt, Licht im sichtbaren Bereich, oder UVLicht abstrahlt.

6. Vorrichtung (1) nach einem der vorhergehenden Ansprüche, **dadurch gekennzeichnet, dass** die Sonde (4) einen Außendurchmesser von maximal 20 bis 30 gauge aufweist.

7. Vorrichtung (1) nach einem der vorhergehenden Ansprüche, **dadurch gekennzeichnet, dass** ein proximaler Bereich (11) des Schaftes als Handstück (30) zur Navigation des Lichtkegels ausgestaltet ist.

8. Vorrichtung (1) nach einem der vorhergehenden Ansprüche, **dadurch gekennzeichnet, dass** im proximalen Bereich des Schaftes (11) eine Halterung (30) angebracht ist, wobei die Halterung (30) de-arretiert und entfernt werden kann.

9. Vorrichtung (1) nach einem der vorhergehenden Ansprüche, **dadurch gekennzeichnet, dass** die Energiequelle (6) eine oder mehrere Batterien, einen oder mehrere Akkus, einen oder mehrere Kondensatoren oder Kombinationen davon umfasst.

10. Vorrichtung (1) nach einem der vorhergehenden Ansprüche, **dadurch gekennzeichnet, dass** die Energiequelle im proximalen Bereich des Schaftes in den Schaft integriert ist oder dass die Energiequelle in einem Gehäuse integriert ist.

11. Vorrichtung (1) nach einem der vorhergehenden Ansprüche, **dadurch gekennzeichnet, dass** die Vorrichtung (1) einen Strombegrenzer aufweist.

12. Vorrichtung (1) nach einem der vorhergehenden Ansprüche, **dadurch gekennzeichnet, dass** die Vorrichtung (1) einen nicht mit dem Schaft verbundenen Instrumentenkanal aufweist, in dem zumindest der distale Bereich des Schaftes rotationsbeweglich geführt wird, wobei der Instrumentenkanal am Schaft von distal nach proximal verläuft und am distalem Ende eine Öffnung für den Austritt des Lichtes aufweist.

13. Vorrichtung (1) nach einem der vorhergehenden Ansprüche, **dadurch gekennzeichnet, dass** die Vorrichtung (1) alle Bestandteile integral umfasst und/oder als Wegwerfartikel ausgebildet ist.

14. Vorrichtung (1) nach einem der vorhergehenden Ansprüche, **dadurch gekennzeichnet, dass** einige oder alle Komponenten a), b), c), d) und e) der Vorrichtung (1) getrennt vorliegen können und zusammensetzbar sind.

15. Vorrichtung (1) nach einem der vorhergehenden Ansprüche, **dadurch gekennzeichnet, dass** das distale Ende des Schafts so ausgebildet ist, dass es beim Überstreichen von Gewebe dieses nicht schädigt.

**Claims**

1. Device (1) for a targeted illumination of an intraocular space of a human or animal eye comprising

   a) a base (2) with a proximal end and a distal end,
   b) a shaft (3), with a proximal end and a distal end,

c) a probe (4), with a proximal end and a distal end,
d) a light source (5) with at least one LED, and
e) an energy source (6) connected to the light source (5),
wherein the shaft is connectable at its distal end to the proximal end of the base, the probe is connectable at its proximal end to the distal end of the base and wherein the light source (5) is positioned at the distal end of the probe (4) and forms the distal end of the probe and is not arranged within the probe (4) or the shaft (3) so that the light emitted by the light source (5) is emitted directly into the eye without being guided via a light guide, **characterised in that** the beam angle of the at least one LED is limited by a reflector or is adjusted by an encapsulation the LED, in particular with a transparent material.

2. Device (1) according to claim 1, **characterised in that** the light source (5) features an underside which is arranged in such a way that an angle $\beta$ of 0° to 90" is formed between the underside of the light source and a longitudinal axis (A) of the shaft.

3. Device (1) according to any one of claims 1 or 2, **characterised in that** the base and probe are integral.

4. Device (1) according to any one of the preceding claims, **characterised in that** the device (1) has an interface, which can be connected to a control instrument with which the wavelength, colour temperature, intensity and/or brightness of the light can be adjusted.

5. Device (1) according to any one of the preceding claims, **characterised in that** the light source comprises at least one LED that emits light with a wavelength in the near, mid or far infrared range, light in the visible range or UV light.

6. Device (1) according to any one of the preceding claims, **characterised in that** the probe (4) has an outer diameter of at most 20 to 30 gauge.

7. Device (1) according to any one of the preceding claims, **characterised in that** a proximal region (11) of the shaft is designed as a handpiece (30) for navigating the light cone.

8. Device (1) according to any one of the preceding claims, **characterised in that** a holder (30) is attached in the proximal region of the shaft (11), wherein the holder (30) can be de-locked and removed.

9. Device (1) according to any one of the preceding claims, **characterised in that** the energy source (6) comprises one or more batteries, one or more accumulators, one or more capacitors or combinations thereof.

10. Device (1) according to any one of the preceding claims, **characterised in that** the energy source is integrated into the shaft in the proximal area of the shaft or that the energy source is integrated into a casing.

11. Device (1) according to any one of the preceding claims, **characterised in that** the device (1) has a current limiter.

12. Device (1) according to any one of the preceding claims, **characterised in that** the device (1) has an instrument channel which is not connected to the shaft and in which at least the distal region of the shaft is rotatably guided, with the instrument channel on the shaft extending from distal to proximal and having an opening at the distal end for the output of the light.

13. Device (1) according to any one of the preceding claims, **characterised in that** the device (1) comprises all components integrally and/or is designed as a disposable article.

14. Device (1) according to any one of the preceding claims, **characterised in that** some or all components a), b), c), d) and e) of the device (1) can be present separately and the device (1) is sectional.

15. Device (1) according to any one of the preceding claims, **characterised in that** the distal end of the shaft is designed in such a way that it does not damage tissue when brushing over it.

**Revendications**

1. Dispositif (1) destiné à l'éclairage ciblé d'un espace intraoculaire d'un œil humain ou animal comprenant

a) un socle (2) avec une extrémité proximale et une extrémité distale,

b) une tige (3), avec une extrémité proximale et une extrémité distale,

c) une sonde (4), avec une extrémité proximale et une extrémité distale,

d) une source de lumière (5) avec au moins une DEL, et

e) une source d'énergie (6) reliée à la source de lumière (5),

dans lequel la tige peut être reliée à son extrémité distale avec l'extrémité proximale du socle, la sonde peut être reliée à son extrémité proximale avec l'extrémité distale du socle, et dans lequel la source de lumière (5) est positionnée à l'extrémité distale de la sonde (4) et forme l'extrémité distale de la sonde et n'est pas agencée à l'intérieur de la sonde (4) ou de la tige (3), de sorte que la lumière émise par la source de lumière (5) est irradiée directement dans l'œil, sans être guidée via un conduit de lumière,

**caractérisé en ce que** l'angle de rayonnement de la au moins une DEL est délimité par un réflecteur ou réglé par une encapsulation de la DEL, en particulier avec un matériau transparent.

2. Dispositif (1) selon la revendication 1, **caractérisé en ce que** la source de lumière (5) présente un côté inférieur, lequel est agencé de sorte qu'un angle $\beta$ de 0° à 90'' soit formé entre le côté inférieur de la source de lumière et l'axe longitudinal (A) de la tige.

3. Dispositif (1) selon l'une des revendications 1 ou **2, caractérisé en ce que** le socle et la sonde sont d'un seul tenant.

4. Dispositif (1) selon l'une des revendications précédentes, **caractérisé en ce que** le dispositif (1) présente une interface qui peut être reliée à un équipement de commande via lequel la longueur d'onde, la température de couleur, l'intensité et/ou la luminosité de la lumière peuvent être réglées.

5. Dispositif (1) selon l'une des revendications précédentes, **caractérisé en ce que** la source lumineuse comprend au moins une DEL qui irradie de la lumière avec une longueur d'onde dans l'infrarouge proche, moyen ou lointain, irradie de la lumière dans la plage visible, ou irradie de la lumière UV.

6. Dispositif (1) selon l'une des revendications précédentes, **caractérisé en ce que** la sonde (4) présente un diamètre extérieur de 20 à 30 gauges au maximum.

7. Dispositif (1) selon l'une des revendications précédentes, **caractérisé en ce qu'**une région proximale (11) de la tige est configurée en tant que pièce à main (30) pour la navigation du cône de lumière.

8. Dispositif (1) selon l'une des revendications précédentes, **caractérisé en ce qu'**un support (30) est monté dans la région proximale de la tige (11), dans lequel le support (30) peut être débloqué et retiré.

9. Dispositif (1) selon l'une des revendications précédentes, **caractérisé en ce que** la source d'énergie (6) comprend une ou plusieurs piles, un ou plusieurs accumulateurs, un ou plusieurs condensateurs ou des combinaisons de ceux-ci.

10. Dispositif (1) selon l'une des revendications précédentes, **caractérisé en ce que** la source d'énergie est intégrée jusque dans la tige dans la région proximale de la tige ou **en ce que** la source d'énergie est intégrée dans un logement.

11. Dispositif (1) selon l'une des revendications précédentes, **caractérisé en ce que** le dispositif (1) présente un limiteur de courant.

12. Dispositif (1) selon l'une des revendications précédentes, **caractérisé en ce que** le dispositif (1) présente un canal instrumental non relié à la tige et dans lequel au moins la région distale de la tige est guidée de façon mobile en rotation, dans lequel le canal instrumental se déroule au niveau de la tige du côté distal vers le côté proximal et présente à l'extrémité distale une ouverture pour la sortie de la lumière.

13. Dispositif (1) selon l'une des revendications précédentes, **caractérisé en ce que** le dispositif (1) comprend toutes les composantes d'un seul tenant et/ou est conçu en tant qu'article jetable.

14. Dispositif (1) selon l'une des revendications précédentes, **caractérisé en ce que** certaines ou toutes les composantes a), b), c), d) et e) du dispositif (1) peuvent se présenter séparément et peuvent être assemblées ensemble.

15. Dispositif (1) selon l'une des revendications précédentes, **caractérisé en ce que** l'extrémité distale de la tige est conçue de manière à ne pas endommager les tissus lorsqu'elle glisse dessus.

Figur 1

EP 3 614 903 B1

Figur 2

70

74

72

73

76

75

Figur 3

EP 3 614 903 B1

19

a)    b)    c)

β

β

5

β

5

5

4    4    4

A    A    A

β =90°    β =45°    β =0°

# Fig. 4

Figur 5

Fig. 6

Figur 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040004846 A1
- DE 102006051736 A1
- EP 2060226 B1
- US 20080208176 A1